Europäisches Patentamt

European Patent Office

Office européen des brevets

(11) Publication number: **0 278 715**
**A2**

# (12) EUROPEAN PATENT APPLICATION

(21) Application number: **88301031.6**

(22) Date of filing: **08.02.88**

(51) Int. Cl.⁴: **A 61 K 45/02**

(30) Priority: **09.02.87 US 12751**

(43) Date of publication of application:
**17.08.88 Bulletin 88/33**

(84) Designated Contracting States:
**AT BE CH DE ES FR GB GR IT LI LU NL SE**

(71) Applicant: **SCHERING CORPORATION**
**2000 Galloping Hill Road**
**Kenilworth, New Jersey 07033 (US)**

(72) Inventor: **Smiles, Kenneth A.**
**44 Hawthorne Lane**
**E. Windsor New Jersey 08520 (US)**

**Peets, Edwin A.**
**160 West 96th Street**
**New York New York 10025 (US)**

**Tanner, Daniel J.**
**1401 55th Street**
**Brooklyn New York 11219 (US)**

(74) Representative: **Ritter, Stephen David et al**
**Mathys & Squire 10 Fleet Street**
**London EC4Y 1AY (GB)**

(54) Use of human gamma interferon for treatment of basal cell carcinoma.

(57) The use of human gamma interferon in treating basal cell carcinoma is described.

EP 0 278 715 A2

## Description

## USE OF HUMAN GAMMA INTERFERON FOR TREATMENT OF BASAL CELL CARCINOMA

This invention relates to a means of treating basal cell carcinoma with human-gamma interferon(hIFN-γ).

Gamma interferon has a number of characteristics known in the art that differentiates it from alpha and beta interferons. Among these differences are antigenic distinctiveness and higher levels of immunoregulatory and antitumor activity. Human gamma interferon may be produced by T-lymphocytes stimulated by mitogens or by antigens to which they are sensitized. It may also be obtained through cloning and expression techniques now well known in the art.

Although the source of the human gamma interferon used in this invention is not critical, it is required that such hIFN-γ is of a high purity material that is not contaminated by cell constituents or cell debris of the interferon-expressing cell. A preferred hIFN-γ used in this invention is produced by recombinant DNA technology and then purified as taught in Japanese Patent Application No. 59-281376, filed December 12, 1984 and the corresponding PCT Patent Application WO 8604067, published July 17, 1986. The purification process comprises adding one or more salts of zinc or copper and polyethyleneimine in the extraction. More particularly, it comprises suspending the culture cells of a recombinant microorganism in a buffer solution containing zinc sulfate, zinc acetate, zinc acetylacetonate and copper sulfate in a range of about 0.5-5mM in the case of zinc salts and 0.01-3mM in the case of copper salts, disrupting the cells, then adding polyethyleneimine to the centrifuged supernatant, e.g. to a final concentration of 0.5-1.1%, and subsequently purifying by a conventional method, e.g. combining several chromatographic methods and dialysis.

Human gamma interferon can be made, for example, by the procedures disclosed in Gray et al, Nature, 295, 503-508 (1982) and Epstein, Nature, 295, 453-454 (1982).

There has been speculation that components of interferon have antitumor properties in humans. Tests with relatively unpurified interferon have been conducted on animals, Gresser, Advan. Cancer Res. 16, 97-140 (1972) and Gresser, Cancer-A Comprehensive Treatise, F. Becher editor, 5, 521-571 (1977), Plenum Press, N.Y. However, there have been no conclusive studies of the effects of hIFN-γ on human basal cell carcinoma cells freshly obtained from patients or by in vivo studies.

This invention relates to a method of treating human basal cell carcinoma, especially cutaneous basal cell carcinoma, characterized by administering to a patient in need of such treatment a sufficient amount of human gamma interferon to be effective as an anti-basal cell carcinoma agent. The human gamma interferon preferably is purified recombinant human gamma interferon. The mode of administration may be parenteral, i.e. intravenous, intramuscular, subcutaneous, intraperitoneal and intralesional.

The last is the preferred method of administration. The treatment normally comprises administration of a plurality of doses of interferon. Typically a dose of at least 0.01 mg of said interferon is administered more than one time per week, normally three times per week, on non-consecutive days, for more than one week.

This invention also relates to the use of human gamma interferon for preparing a pharmaceutical composition for treating basal cell carcinoma.

This invention also relates to human gamma interferon for use as a medicament for curing basal cell carcinoma.

In order to investigate and determine the effect of human gamma interferon on basal cell carcinoma, a study to investigate two different doses of gamma interferon was carried out. The study was an open label, third party blinded, study of the efficacy and safety of two different intralesional doses of gamma interferon in the treatment of a basal cell carcinoma. Two study centers each enrolled thirteen to sixteen patients with approximately half of the patients per center receiving one of the two doses. Of the patients assigned to each dose at a given center, approximately half had the clinical diagnosis of noduloulcerative basal cell carcinoma, and half , superficial basal cell carcinoma. The patients were randomly assigned to the dosage regimens.

The dosage regimens were as follows:

A. 0.05 mg purified human gamma interferon administered three times per week, on non-consecutive days for three weeks.

B. 0.01 mg purified human gamma interferon administered three times per week, on non-consecutive days for three weeks.

The drug was supplied in vials containing the dosage to be injected i.e. either 0.05 mg or 0.01 mg of purified human gamma interferon. The vials were reconstituted with a pharmaceutically acceptable carrier comprising one milliliter of sterile water containing no preservatives for injection.

The presence of a basal cell carcinoma in each patient prior to the start of treatment was confirmed by biopsy of each lesion. During the treatment period, the treated lesions were measured, photographed, and morphological changes evaluated. At the last follow-up visit (post-treatment week 12), the test side was rebiopsied (excisional) to see if any basal cell carcinoma remained.

The results were as follows:

Of the 14 tumors injected with 0.01mg doses, one tumor was cured. Of the 14 tumors injected with 0.05mg doses seven were cured. Side effects were infrequent and generally mild. Thus, it is apparent that purified human gamma interferon is effective for treating basal cell carcinoma.

The dosage amounts and dosage regimen used for each patient is determined, in the judgment of the clinician, taking into consideration the severity of the disease, by the size and age of the patient and the condition of the patient as well as other criteria.

Generally more than about 0.01mg, preferably more than about 0.05mg of purified hIFN-γ is administered three times a week on alternate days, for three weeks. The highest dosage of the interferon is an amount which is effective but not toxic. If additional courses of treatment are required, it is determined by the clinician. The preferred mode of administration is intralesional administration.

## Claims

1. The use of human gamma interferon for preparing a pharmaceutical composition for treating basal cell carcinoma.

2. The use of claim 1 characterized by said human gamma interferon being purified recombinant human gamma interferon.

3. The use of either claim 1 or claim 2 above characterized by administering a dose of at least 0.01 mg of said interferon.

4. The use of any of claims 1-3 above characterized by administering a plurality of doses of said interferon.

5. The use of any of claims 1-4 above characterized by administering said dose of said interferon more than one time per week.

6. The use of any of claims 1-5 above characterized by administering said dose of said interferon on non-consecutive days.

7. The use of any of claims 1-6 above characterized by administering said doses of said interferon over more than one week.

8. The use of any of claims 1-7 above characterized by administering said dose of said interferon by injection.

9. The use of any of claims 1-8 above characterized by said interferon being injected intralesionally.

10. The use of any of claims 1-9 above characterized by said dose comprising said interferon and a pharmaceutically acceptable carrier.

11. The use of any of claims 1-10 above characterized by said pharmaceutically acceptable carrier comprising sterile water.

12. Human gamma interferon for use as a medicament for curing basal cell carcinoma.

13. A method of treating basal cell carcinomas characterized by administering to a patient in need of such treatment, a sufficient amount of human gamma interferon to be effective in treating basal cell carcinoma.